(19) Europäisches Patentamt
European Patent Office
Office européen des brevets

(11) Publication number : **0 467 844 A2**

# (12) EUROPEAN PATENT APPLICATION

(21) Application number : **91810551.1**

(51) Int. Cl.$^5$ : **C09K 15/18**

(22) Date of filing : **10.07.91**

(30) Priority : **19.07.90 US 555289**

(43) Date of publication of application :
**22.01.92 Bulletin 92/04**

(84) Designated Contracting States :
**BE DE ES FR GB IT NL**

(71) Applicant : **CIBA-GEIGY AG**
**Klybeckstrasse 141**
**CH-4002 Basel (CH)**

(72) Inventor : **Gatechair, Leslie R.**
**Rural Route 2, Box 60**
**Katonah, NY 10536 (US)**
Inventor : **Klemchuk, Peter P.**
**148 Upland Road**
**Yorktown Heights, NY 10598 (US)**
Inventor : **Hyun, James L.**
**6 Kevin Drive**
**Danbury, CT 06811 (US)**

(54) **Prevention of oxidation in petrochemical extraction solvents.**

(57) A wide variety of hindered amine compounds are effective as antioxidant stabilizers to prevent the oxidation of liquid hydrocarbons and of oxygenated compounds, such as sulfolane or tetraethylene glycol, used as extraction solvents in the petroleum industry.

EP 0 467 844 A2

This invention pertains to a method of preventing the oxidation of liquid hydrocarbons and of oxygenated solvents used in the petroleum industry by the use of hindered amine stabilizers.

Organic compounds including liquid hydrocarbons and various oxygenated solvents are subject to oxidative and/or thermal degradation. The results of such unwanted oxidative reactions may be the loss of desirable properties, formation of color and/or odors and the presence of unwanted and corrosive by-products. A variety of liquid media are subject to such unwanted oxidation including saturated hydrocarbons, especially if they contain branched chains having tertiary carbon atoms.

T. Wheeler, Handbook of Petroleum Refinery Processes, R. A. Meyers, Edit. McGraw-Hill, New York, 1968, teaches the use of sulfolane to aid in the separation of $C_6$-$C_9$ aromatic hydrocarbons from aliphatic hydrocarbons having a similar boiling range, based on the selective solubility of the aromatic hydrocarbons in the sulfolane.

Somekh and Friedlander (Hydrocarbon Processing, 1969, pp. 127-130 and Refining Petroleum for Chemicals, 1970, pp. 228-241) teach that glycols are effective selective solvents for extraction of aromatics from gasoline fractions during refining of petroleum. It is stated that small changes in solubility of aromatics or aliphatics in the separation or extraction solvent can have a dramatic effect on the efficiency of the entire process. Such changes can occur by adding a cosolvent such as water or by contamination of the solvent with heavy residues carried with the feedstock which effect the polarity of the solution. In addition, polarity can be affected by oxidation or hydrolysis of the solvent or rafinate resulting in a change in the partitioning of the aromatics and aliphatics in the extraction solvent.

Other saturated organic compounds, such as ethers, i.e. diethyl ether, tetraethylene glycol, are also subject to oxidation with the formation of peroxides which may result in fires and explosions. Such chemicals require special handling.

Some organic materials are solid at ambient temperature, but liquefy at slightly higher temperatures. Hydrocarbon waxes exemplify such materials which are subject to oxidation in the liquid state. Furfural is used to dissolve and remove paraffinic waxes from refinery streams.

In other cases, the organic material may be a solid which is dissolved in a solvent where it can be subject to oxidation.

In each of the above scenarios, the instant hindered amine compounds provide antioxidant stabilization protection to the organic material subject to oxidative or thermal degradation.

It is surmised that the instant hindered amine stabilizers act to prevent the oxidation at carbon sites in the organic molecules having labile hydrogen atoms which are amenable to abstraction. Following abstraction of the hydrogen, oxygen may react with the site. Thus, an alkane is converted to a peroxide, hydroperoxide or indirectly to an alcohol while an aldehyde may be converted to an acid, etc. Such reactions are free radical in nature and the addition of a free radical chain stopping agent, such as the instant hindered amine compounds, acts to inhibit the chain propagation and to protect the site from oxidation.

United States Patent Nos. 3,644,278 and 3,778,464 describe the use of substituted hydroxylamine antioxidants in hydrocarbon fuels, kerosene, oils, paraffin, animal and vegetable oils. The antioxidants disclosed include dibenzylhydroxylamines, triphenylmethylhydroxylamine and triazine substituted hydroxylamines. These patents also teach the use of the oxygen uptake method for screening the relative activity of antioxidants on various organic substrates dissolved in chlorobenzene. Typical substrates include tetralin, cyclohexane, lard, mineral oil and polymeric substances. No hindered amine antioxidants are disclosed in these references.

There is still a need of effective stabilizers against oxidation of liquid hydrocarbons and oxigenated compounds.

The instant invention pertains to a process for preventing the oxidation of a liquid organic substrate subject to oxidative or thermal degradation at temperatures below 200°C, which process comprises incorporating into said substrate a compound having present a moiety of formula I

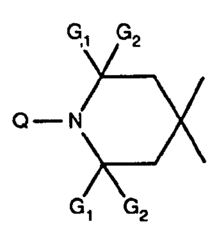

(I)

wherein $G_1$ and $G_2$ are independently alkyl of 1 to 4 carbon atoms, or $G_1$ and $G_2$ together are pentamethylene, Q is hydrogen, oxyl, hydroxy, alkyl of 1 to 18 carbon atoms, alkenyl of 3 to 18 carbon atoms, alkanoyl of 1 to

18 carbon atoms, alkenoyl of 3 to 18 carbon atoms, phenylalkyl of 7 to 9 carbon atoms, alkanoyloxy of 2 to 18 carbon atoms, alkoxy of 1 to 18 carbon atoms, cycloalkoxy of 5 to 12 carbon atoms, alkenyloxy of 2 to 18 carbon atoms, cycloalkenyloxy of 5 to 12 carbon atoms, phenylalkoxy of 7 to 15 carbon atoms, aryloxy of 6 to 10 carbon atoms or $R_1$NHCOO- where $R_1$ is hydrogen, alkyl of 1 to 12 carbon atoms or phenyl.

Preferably $G_1$ and $G_2$ are each methyl.

Preferably Q is hydrogen, oxyl, hydroxy, alkyl of 1 to 8 carbon atoms, allyl, alkanoyl of 1 to 8 carbon atoms, alkenoyl of 3 to 4 carbon atoms, benzyl, alkanoyloxy of 2 to 8 carbon atoms, alkoxy of 1 to 12 carbon atoms, cyclohexyloxy, α-methylbenzyloxy, carbamoyloxy, phenylcarbamoyloxy or alkylcarbamoyloxy of 2 to 9 carbon atoms.

Most preferably Q is hydrogen, oxyl, hydroxy, alkyl of 1 to 4 carbon atoms, allyl, alkanoyl of 2 to 4 carbon atoms, benzyl, acetoxy, alkoxy of 1 to 8 carbon atoms, cyclohexyloxy, α-methylbenzyloxy or carbamoyloxy.

Preferably, the instant compounds are of formula II

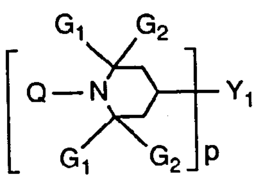

(II)

wherein $G_1$, $G_2$ and Q are defined as described above,
p is 1 or 2,
when p is 1, $Y_1$ is hydrogen, oxyl, hydroxyl or $-OY_2$
where $Y_2$ is alkanoyl of 2 to 19 carbon atoms, benzoyl or benzoyl substituted by alkyl of 1 to 4 carbon atoms, or
when p is 2, $Y_1$ is $-OCO-Y_3-COO-$
where $Y_3$ is a straight or branched chain alkylene of 2 to 16 carbon atoms.

Examples for compounds containing a moiety of formulae I are those of formulae 1, 2, 3, 4.1, 4.2, 4.3, 5 and 6.

a) Compounds of the formula 1

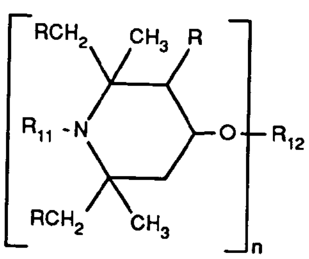

(1),

in which n is a number from 1 to 4, preferably 1 or 2, R is hydrogen or methyl, $R_{11}$ is hydrogen, oxyl, hydroxyl, $C_1$-$C_{12}$alkyl, $C_3$-$C_8$alkenyl, $C_3$-$C_8$alkynyl, $C_7$-$C_{12}$aralkyl, $C_1$-$C_{18}$alkoxy, $C_5$-$C_8$cycloalkoxy, $C_7$-$C_9$phenylalkoxy, $C_1$-$C_8$alkanoyl, $C_3$-$C_5$alkenoyl, $C_1$-$C_{18}$alkanoyloxy, benzyloxy, glycidyl or a $-CH_2CH(OH)-Z$ group, in which Z is hydrogen, methyl or phenyl, $R_{11}$ preferably being H, $C_1$-$C_4$alkyl, allyl, benzyl, acetyl or acryloyl, and $R_{12}$, if n is 1, being hydrogen, $C_1$-$C_{18}$alkyl which is uninterrupted or interrupted by one or more oxygen atoms, cyanoethyl, benzyl, glycidyl, a monovalent radical of an aliphatic, cycloaliphatic, araliphatic, unsaturated or aromatic carboxylic acid, carbamic acid or phosphorus-containing acid or a monovalent silyl radical, preferably a radical of an aliphatic carboxylic acid having from 2 to 18 carbon atoms, of a cycloaliphatic carboxylic acid having from 7 to 15 carbon atoms, of an α,β-unsaturated carboxylic acid having from 3 to 5 carbon atoms or of an aromatic carboxylic acid having from 7 to 15 carbon atoms, $R_{12}$ being, if n is 2, $C_1$-$C_{12}$alkylene, $C_4$-$C_{12}$alkenylene, xylylene, a divalent radical of an aliphatic, cycloaliphatic, araliphatic or aromatic dicarboxylic acid, dicarbamic acid or phosphorus-containing acid, or a divalent silyl radical, preferably a radical of an aliphatic dicarboxylic acid having from 2 to 36 carbon atoms, of a cycloaliphatic or aromatic dicarboxylic acid having from 8 to 14 carbon atoms or of an aliphatic, cycloaliphatic or aromatic dicarbamic acid having from 8 to 14 carbon atoms,

$R_{12}$ being, if n is 3, a trivalent radical of aliphatic, cycloaliphatic or aromatic tricarboxylic acid, of an aromatic tricarbamic acid or of a phosphorus-containing acid, or a trivalent silyl radical, and $R_{12}$ being, if n is 4, a tetravalent radical of an aliphatic, cycloaliphatic or aromatic tetracarboxylic acid.

If any substituents are $C_1$-$C_{12}$alkyl, they are, for example, methyl, ethyl, n-propyl, n-butyl, sec-butyl, tert-butyl, n-hexyl, n-octyl, 2-ethylhexyl, n-nonyl, n-decyl, n-undecyl or n-dodecyl.

$C_1$-$C_{18}$Alkyl $R_{11}$ or $R_{12}$ may be, for example, the abovementioned groups and additionally, for example, n-tridecyl, n-tetradecyl, n-hexadecyl or n-octadecyl.

$C_3$-$C_8$Alkenyl $R_{11}$ may be, for example, 1-propenyl, allyl, methallyl, 2-butenyl, 2-pentenyl, 2-hexenyl, 2-octenyl or 4-tert-butyl-2-butenyl.

$C_3$-$C_8$Alkynyl $R_{11}$ is preferably propargyl.

$C_7$-$C_{12}$Aralkyl $R_{11}$ is in particular phenethyl and especially benzyl.

$C_1$-$C_8$Alkanoyl $R_{11}$ is, for example, formyl, propionyl, butyryl or octanyl, but preferably acetyl and $C_3$-$C_5$alkenoyl $R_{11}$ is in particular acryloyl.

A monovalent carboxylic acid radical $R_{12}$ is, for example, an acetic acid, caproic acid, stearic acid, acrylic acid, methacrylic acid, benzoic acid or β-(3,5-di-tert-butyl-4-hydroxyphenyl)propionic acid radical.

A divalent dicarboxylic acid radical $R_{12}$ is, for example, a malonic acid, succinic acid, glutaric acid, adipic acid, suberic acid, sebacic acid, maleic acid, itaconic acid, phthalic acid, dibutylmalonic acid, dibenzylmalonic acid, butyl(3,5-di-tert-butyl-4-hydroxybenzyl)malonic acid or bicycloheptenedicarboxylic acid radical.

A trivalent tricarboxylic acid $R_{12}$ is, for example, a trimellitic acid, citric acid or nitrilotriacetic acid radical.

A tetravalent tetracarboxylic acid radical $R_{12}$ is, for example, the tetravalent radical of butane-1,2,3,4-tetracarboxylic acid or of pyromellitic acid.

A divalent dicarbamic acid radical $R_{12}$ is, for example, a hexamethylenedicarbamic acid or 2,4-tolylenedicarbamic acid radical.

Preference is given to compounds of the formula 1 in which R is hydrogen, $R_{11}$ is hydrogen or methyl, n is 2, and $R_{12}$ is the diacyl radical of an aliphatic dicarboxylic acid having from 4 to 12 carbon atoms.

The following compounds are examples of polyalkylpiperidine compounds of this class:

1) 4-hydroxy-2,2,6,6-tetramethylpiperidine
2) 1-allyl-4-hydroxy-2,2,6,6-tetramethylpiperidine
3) 1-benzyl-4-hydroxy-2,2,6,6-tetramethylpiperidine
4) 1-(4-tert-butyl-2-butenyl)-4-hydroxy-2,2,6,6-tetramethylpiperidine
5) 4-stearoyloxy-2,2,6,6-tetramethylpiperidine
6) 1-ethyl-4-salicyloyloxy-2,2,6,6-tetramethylpiperidine
7) 4-methacryloyloxy-1,2,2,6,6-pentamethylpiperidine
8) 1,2,2,6,6-pentamethylpiperidin-4-yl β-(3,5-di-tert-butyl-4-hydroxyphenyl)propionat
9) di(1-benzyl-2,2,6,6-tetramethylpiperidin-4-yl) maleinate
10) di(2,2,6,6-tetramethylpiperidin-4-yl) succinate
11) di(2,2,6,6-tetramethylpiperidin-4-yl) glutarate
12) di(2,2,6,6-tetramethylpiperidin-4-yl) adipate
13) di(2,2,6,6-tetramethylpiperidin-4-yl) sebacate
14) di(1,2,2,6,6-pentamethylpiperidin-4-yl) sebacate
15) di(1,2,3,6-tetramethyl-2,6-diethyl-piperidin-4-yl) sebacate
16) di(1-allyl-2,2,6,6-tetramethylpiperidin-4-yl) phthalate
17) 1-hydroxy-4-β-cyanoethyloxy-2,2,6,6-tetramethylpiperidine
18) 1-acetyl-2,2,6,6-tetramethylpiperidin-4-yl acetate
19) tri(2,2,6,6-tetramethylpiperidin-4-yl) trimellitate
20) 1-acryloyl-4-benzyloxy-2,2,6,6-tetramethylpiperidine
21) di(2,2,6,6-tetramethylpiperidin-4-yl) diethylmalonate
22) di(1,2,2,6,6-pentamethylpiperidin-4-yl) dibutylmalonate
23) di(1,2,2,6,6-pentamethylpiperidin-4-yl) butyl(3,5-di-tert-butyl-4-hydroxybenzyl)malonate
24) di(1-octyloxy-2,2,6,6-tetramethylpiperidin-4-yl) sebacate
25) di(1-cyclohexyloxy-2,2,6,6-tetramethylpiperidin-4-yl) sebacate
26) hexan-1′,6′-bis-(4-carbamoyloxy-1-n-butyl-2,2,6,6-tetramethylpiperidine)
27) toluene-2′,4′-bis-(4-carbamoyloxy-1-n-propyl-2,2,6,6-tetramethylpiperidine)
28) dimethylbis(2,2,6,6-tetramethylpiperidin-4-oxy)silane
29) phenyltris(2,2,6,6-tetramethylpiperidin-4-oxy)silane
30) tris(1-propyl-2,2,6,6-tetramethylpiperidin-4-yl) phosphite
31) tris(1-propyl-2,2,6,6-tetramethylpiperidin-4-yl) phosphate
32) phenyl[bis-(1,2,2,6,6-pentamethylpiperidin-4-yl)] phosphonate

33) 4-hydroxy-1,2,2,6,6-pentamethylpiperidine
34) 4-hydroxy-N-hydroxyethyl-2,2,6,6-tetramethylpiperidine
35) 4-hydroxy-N-(2-hydroxypropyl)-2,2,6,6-tetramethylpiperidine
36) 1-glycidyl-4-hydroxy-2,2,6,6-tetramethylpiperidine

b) Compounds of the formula (2)

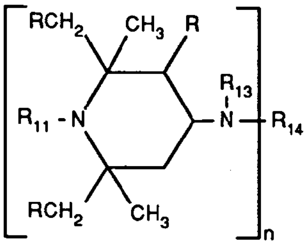

(2)

in which n is the number 1 or 2, R and $R_{11}$ are as defined under a), $R_{13}$ is hydrogen, $C_1$-$C_{12}$alkyl, $C_2$-$C_5$hydroxyalkyl, $C_5$-$C_7$cycloalkyl $C_7$-$C_8$aralkyl, $C_2$-$C_{18}$alkanoyl, $C_3$-$C_5$alkenoyl, benzoyl or a group of the formula

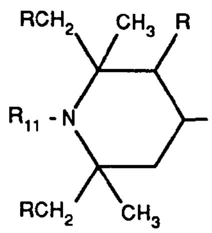

and $R_{14}$, if n is 1, is hydrogen, $C_1$-$C_{18}$alkyl, $C_3$-$C_8$alkenyl, $C_5$-$C_7$cycloalkyl, $C_1$-$C_4$alkyl which is substituted by a hydroxyl, cyano, alkoxycarbonyl or carbamide group, glycidyl, a group of the formula $-CH_2-CH(OH)-Z$ or of the formula -CONH-Z in which Z is hydrogen, methyl or phenyl; if n is 2, $R_{14}$ is $C_2$-$C_{12}$alkylene, $C_6$-$C_{12}$arylene, xylylene, a $-CH_2-CH(OH)-CH_2-$ group or a $-CH_2-CH(OH)-CH_2-O-D-O-$ group, in which D is $C_2$-$C_{10}$alkylene, $C_6$-$C_{15}$arylene, $C_6$-$C_{12}$cycloalkylene or, as long as $R_{13}$ is not alkanoyl, alkenoyl or benzoyl, $R_{14}$ may alternatively be a divalent radical of an aliphatic, cycloaliphatic or aromatic dicarboxylic acid or dicarbamic acid or alternatively the -CO- group, or $R_{13}$ and $R_{14}$ together, if n is 1, may be the divalent radical of a aliphatic, cycloaliphatic or aromatic 1,2- or 1,3-dicarboxylic acid.

Any $C_1$-$C_{12}$ or $C_1$-$C_{18}$alkyl substituents are as defined under a).

Any $C_5$-$C_7$cycloalkyl substituents are, in particular, cyclohexyl.

$C_7$-$C_8$Aralkyl $R_{13}$ is, in particular, phenylethyl or especially benzyl. $C_2$-$C_5$Hydroxyalkyl $R_{13}$ is, in particular, 2-hydroxyethyl or 2-hydroxypropyl.

$C_2$-$C_{18}$Alkanoyl $R_{13}$ is, for example, propionyl, butyryl, octanoyl, dodecanoyl, hexadecanoyl or octadecanoyl, but preferably acetyl and $C_3$-$C_5$alkenoyl $R_{13}$ is, in particular, acryloyl.

$C_2$-$C_8$Alkenyl $R_{14}$ is, for example, allyl, methallyl, 2-butenyl, 2-pentenyl, 2-hexenyl or 2-octenyl.

Hydroxyl-, cyano-, alkoxycarbonyl- or carbamide-substituted $C_1$-$C_4$alkyl $R_{14}$ may be, for example, 2-hydroxyethyl, 2-hydroxypropyl, 2-cyanoethyl, methoxycarbonylmethyl, 2-ethoxycarbonylethyl, 2-aminocarbonylpropyl or 2-(dimethylaminocarbonyl)ethyl.

Any $C_2$-$C_{12}$alkylene substituents are, for example, ethylene, propylene, 2,2-dimethylpropylene, tetramethylene, hexamethylene, octamethylene, decamethylene or dodecamethylene.

Any $C_6$-$C_{15}$arylene substituents are, for example, o-, m- or p-phenylene, 1,4-naphthylene or 4,4'-diphenylene.

$C_6$-$C_{12}$Cycloalkylene D is, in particular, cyclohexylene.

Preferred compounds of the formula 2 are those in which n is 1 or 2, R is hydrogen, $R_{11}$ is hydrogen or methyl, $R_{13}$ is hydrogen, $C_1$-$C_{12}$alkyl or a group of the formula

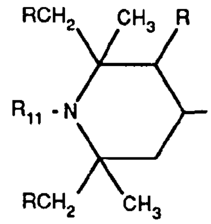

and $R_{14}$ is, in the case where n = 1, hydrogen or $C_1$-$C_{12}$alkyl, and, in the case where n = 2, is $C_2$-$C_8$alkylene.

The following are examples of polyakylpiperidine compounds of this class:

37) N,N′-bis(2,2,6,6-tetramethylpiperidin-4-yl)hexamethylene-1,6-diamine
38) N,N′-bis(2,2,6,6-tetramethylpiperidin-4-yl)hexamethylene-1,6-diacetamide
39) bis(2,2,6,6-tetramethylpiperidin-4-yl)amine
40) 4-benzoylamino-2,2,6,6-tetramethylpiperidine
41) N,N′-bis(2,2,6,6-tetramethylpiperidin-4-yl)-N,N′-dibutyladipamide
42) N,N′-bis(2,2,6,6-tetramethylpiperidin-4-yl)-N,N′-dicyclohexyl-2-hydroxypropylen-1,3-diamine
43) N,N′-bis(2,2,6,6-tetramethylpiperidin-4-yl)-p-xylylenediamine
44) N,N′-bis(2,2,6,6-tetramethylpiperidin-4-yl)-succindiamide
45) di(2,2,6,6-tetramethylpiperidin-4-yl) N-(2,2,6,6-Tetramethylpiperidin-4-yl)-β-aminodipropionate
46) The compound of the formula

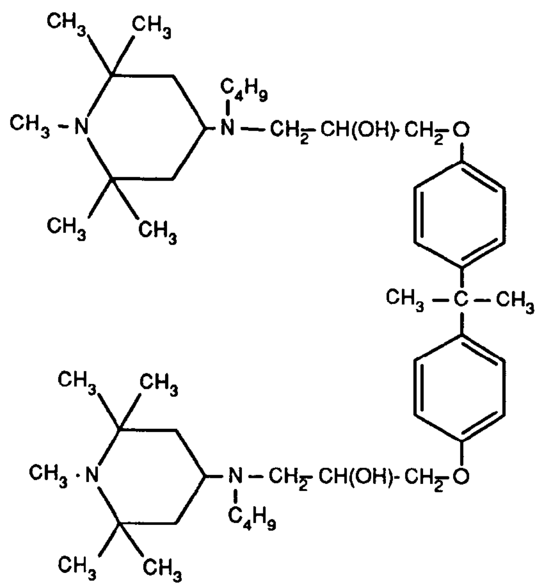

47) 4-(bis-2-hydroxyethyl-amino)-1,2,2,6,6-pentamethylpiperidine
48) 4-(3-Methyl-4-hydroxy-5-tert-butyl-benzamido)-2,2,6,6-tetramethylpiperidine
49) 4-methacrylamido-1,2,2,6,6-pentamethylpiperidine

c) Compounds of the formula (3)

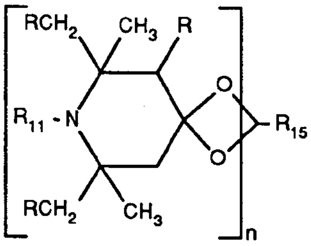

(3)

in which n is the number 1 or 2, R and $R_{11}$ are as defined under a), and $R_{15}$, if n is 1, is $C_2$-$C_8$alkylene or -hydroxyalkylene or $C_4$-$C_{22}$acyloxyalkylene, and, if n is 2, is the $(-CH_2)_2C(CH_2-)_2$ group.

$C_2$-$C_8$Alkylene or -hydroxyalkylene $R_{15}$ is, for example, ethylene, 1-methylethylene, propylene, 2-ethylpropylene or 2-ethyl-2-hydroxymethylpropylene.

$C_4$-$C_{22}$Acyloxyalkylene $R_{15}$ is, for example, 2-ethyl-2-acetoxymethylpropylene.

The following are examples of polyakylpiperidine compounds of this class:

50) 9-aza-8,8,10,10-tetramethyl-1,5-dioxaspiro[5.5]undecane

51) 9-aza-8,8,10,10-tetramethyl-3-ethyl-1,5-dioxaspiro[5.5]undecane

52) 8-aza-2,7,7,8,9,9-hexamethyl-1,4-dioxaspiro[4.5]decane

53) 9-aza-3-hydroxymethyl-3-ethyl-8,8,9,10,10-pentamethyl-1,5-dioxaspiro[5.5]undecane

54) 9-aza-3-ethyl-3-acetoxymethyl-9-acetyl-8,8,10,10-tetramethyl-1,5-dioxaspiro-[5.5]undecane

55) 2,2,6,6-tetramethylpiperidine-4-spiro-2′-(1′,3′-dioxane)-5′-spiro-5″-(1″,3″-dioxane)-2″-spiro-4‴-(2‴,2‴,6‴,6‴- tetramethylpiperidine).

d) Compounds of the formulae 4.1, 4.2 and 4.3

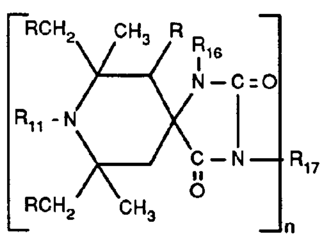

(4.1)

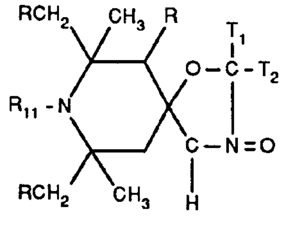

(4.2)

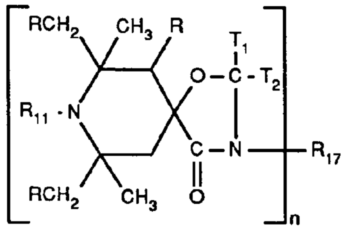

(4.3)

in which n is the number 1 or 2, R and $R_{11}$ are as defined under a), $R_{16}$ is hydrogen, $C_1$-$C_{12}$alkyl, allyl, benzyl, glycidyl or $C_2$-$C_6$alkoxyalkyl, and $R_{17}$, if n is 1, is hydrogen, $C_1$-$C_{12}$alkyl, $C_3$-$C_5$alkenyl, $C_7$-$C_9$aralkyl, $C_5$-$C_7$cycloalkyl, $C_2$-$C_4$hydroxyalkyl, $C_2$-$C_6$alkoxyalkyl, $C_6$-$C_{10}$aryl, glycidyl or a group of the formula $-(CH_2)_p-COO-Q$ or of the formula $-(CH_2)_p-O-CO-Q$, in which p is 1 or 2 and Q is $C_1$-$C_4$alkyl or phenyl, and, if n is 2, $R_{17}$ is $C_2$-$C_{12}$alkylene, $C_4$-$C_{12}$akenylene, $C_6$-$C_{12}$arylene, a $-CH_2-CH(OH)-CH_2-O-D-O-CH_2-CH(OH)-CH_2-$ group in which D is $C_2$-$C_{10}$alkylene, $C_6$-$C_{15}$arylene, $C_6$-$C_{12}$cycloalkylene, or a $-CH_2CH(OZ')CH_2-(OCH_2-CH(OZ')CH_2)_2-$ group in which Z' is hydrogen, $C_1$-$C_{18}$alkyl, allyl, benzyl, $C_2$-$C_{12}$alkanoyl or benzoyl, $T_1$ and $T_2$, independently of one another, are hydrogen, $C_1$-$C_{18}$alkyl or $C_6$-$C_{10}$aryl or $C_7$-$C_9$aralkyl, each of which is unsubstituted or substituted by halogen or $C_1$-$C_4$alkyl, or $T_1$ and $T_2$, together with the carbon atom connecting them, form a $C_5$-$C_{12}$cycloalkane ring.

Any $C_1$-$C_{12}$alkyl substituents are, for example, methyl, ethyl, n-propyl, n-butyl, sec-butyl, tert-butyl, n-hexyl, n-octyl, 2-ethylhexyl, n-nonyl, n-decyl, n-undecyl or n-dodecyl.

Any $C_1$-$C_{18}$alkyl substituents may be, for example, the abovementioned groups and in addition, for example, n-tridecyl, n-tetradecyl, n-hexadecyl or n-octadecyl.

Any $C_2$-$C_6$alkoxyalkyl substituents are, for example, methoxymethyl, ethoxymethyl, propoxymethyl, tert-butoxymethyl, ethoxyethyl, ethoxypropyl, n-butoxyethyl, tert-butoxyethyl, isopropoxyethyl or propoxypropyl.

$C_3$-$C_5$Alkenyl $R_{17}$ is, for example, 1-propenyl, allyl, methallyl, 2-butenyl or 2-pentenyl.

$C_7$-$C_9$Aralkyl $R_{17}$, $T_1$ and $T_2$ are, in particular, phenethyl or especially benzyl. If $T_1$ and $T_2$, together with the carbon atom, form a cycloalkyl ring, it may, for example, be a cyclopentane, cyclohexane, cyclooctane or cyclododecane ring.

$C_2$-$C_4$Hydroxyalkyl $R_{17}$ is, for example, 2-hydroxyethyl, 2-hydroxypropyl, 2-hydroxybutyl or 4-hydroxybutyl.

$C_6$-$C_{10}$Aryl $R_{17}$, $T_1$ and $T_2$ are, in particular, phenyl, α- or β-naphthyl, which are unsubstituted or substituted by halogen or $C_1$-$C_4$alkyl.

$C_2$-$C_{12}$Alkylene $R_{17}$ is, for example, ethylene, propylene, 2,2-dimethylpropylene, tetramethylene, hexamethylene, octamethylene, decamethylene or dodecamethylene.

$C_4$-$C_{12}$Alkenylene $R_{17}$ is, in particular, 2-butenylene, 2-pentenylene or 3-hexenylene.

$C_6$-$C_{12}$Arylene $R_{17}$ is, for example, o-, m- or p-phenylene, 1,4-naphthylene or 4,4'-diphenylene.

$C_2$-$C_{12}$Alkanoyl Z' is, for example, propionyl, butyryl, octanoyl or dodecanoyl, but preferably acetyl.

$C_2$-$C_{10}$Alkylene, $C_6$-$C_{15}$arylene or $C_6$-$C_{12}$cycloalkylene D is as defined under b).

The following are examples of polyalkylpiperidine compounds of this class:

56) 3-benzyl-1,3,8-triaza-7,7,9,9-tetramethylspiro[4.5]decane-2,4-dione

57) 3-n-octyl-1,3,8-triaza-7,7,9,9-tetramethylspiro[4.5]decane-2,4-dione

58) 3-allyl-1,3,8-triaza-1,7,7,9,9-pentamethylspiro[4.5]decane-2,4-dione

59) 3-glycidyl-1,3,8-triaza-7,7,8,9,9-pentamethylspiro[4.5]decane-2,4-dione

60) 1,3,7,7,8,9,9-heptamethyl-1,3,8-triazaspiro[4.5]decane-2,4-dione

61) 2-iso-propyl-7,7,9,9-tetramethyl-1-oxa-3,8-diaza-4-oxospiro[4.5]decane

62) 2,2-dibutyl-7,7,9,9-tetramethyl-1-oxa-3,8-diaza-4-oxospiro[4.5]decane

63) 2,2,4,4-tetramethyl-7-oxa-3,20-diaza-21-oxodispiro[5.1.11.2]heneicosane

64) 2-butyl-7,7,9,9-tetramethyl-1-oxa-4,8-diaza-3-oxospiro[4,5]decane

65) 8-acetyl-3-dodecyl-1,3,8-triaza-7,7,9,9-tetramethylspiro[4,5]-decane-2,4-dione

or the compounds of the following formulae:

66) 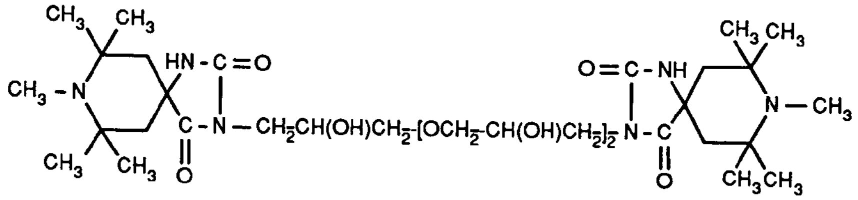

67) 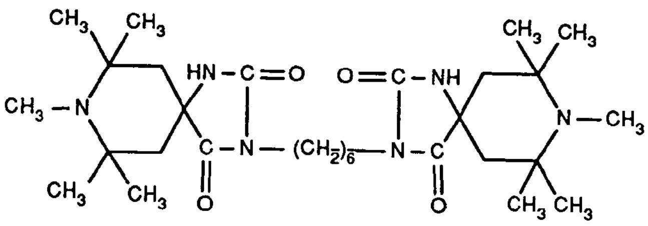

68) 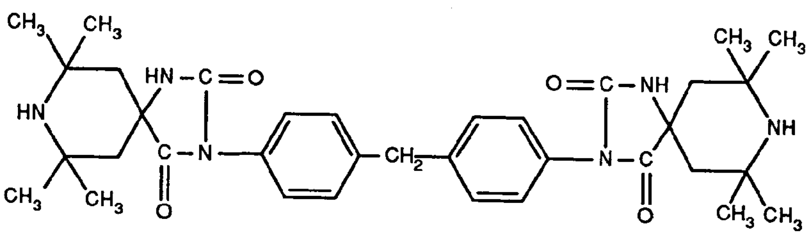

69) 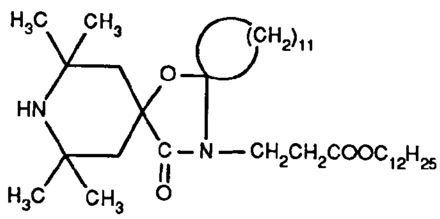

e) Compounds of the formula 5

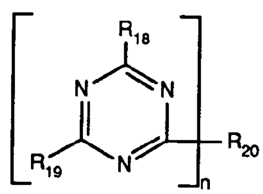 (5),

in which n is the number 1 or 2 and $R_{18}$ is a group of the formula

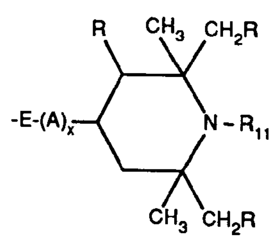

in which R and $R_{11}$ are as defined under a), E is -O- or $-NR_{11}-$, A is $C_2$-$C_6$alkylene or $-(CH_2)_3-O-$, x is the number 0 or 1, $R_{19}$ is identical with $R_{18}$ or is one of the groups $-NR_{21}R_{22}$, $-OR_{23}$, $-NHCH_2OR_{23}$ or $-N(CH_2OR_{23})_2$, $R_{20}$, if n is 1, is identical with $R_{18}$ or $R_{19}$ and, if n is 2, is an -E-B-E- group in which B is $C_2$-$C_6$alkylene which is uninterrupted or is interrupted by $-N(R_{21})-$, $R_{21}$ is $C_1$-$C_{12}$alkyl, cyclohexyl, benzyl or $C_1$-$C_4$hydroxyalkyl or is a group of the formula

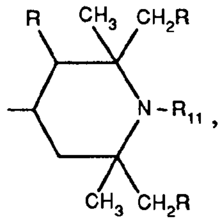

$R_{22}$ is $C_1$-$C_{12}$alkyl, cyclohexyl, benzyl or $C_1$-$C_4$hydroxyalkyl, and $R_{23}$ is hydrogen, $C_1$-$C_{12}$alkyl or phenyl, or $R_{21}$ and $R_{22}$ together are $C_4$-$C_5$alkylene or -oxaalkylene, for example

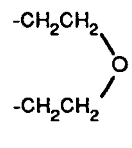

or a group of the formula

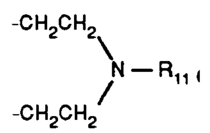

or alternatively $R_{21}$ and $R_{22}$ are each a group of the formula

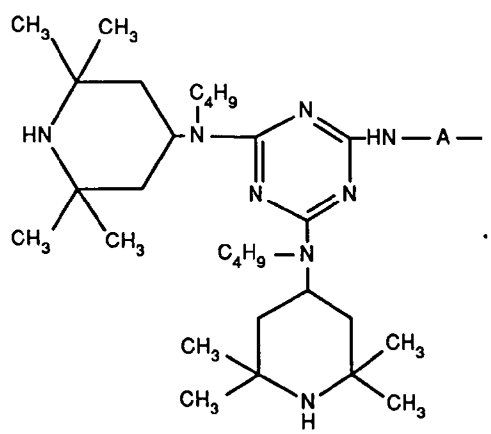

Any $C_1$-$C_{12}$alkyl substituents are, for example, methyl, ethyl, n-propyl, n-butyl, sec-butyl, tert-butyl, n-hexyl, n-octyl, 2-ethylhexyl, n-nonyl, n-decyl, n-undecyl, or n-dodecyl.

Any $C_1$-$C_4$hydroxyalkyl substituents are, for example, 2-hydroxyethyl, 2-hydroxypropyl, 3-hydroxypropyl, 2-hydroxybutyl or 4-hydroxybutyl.

$C_2$-$C_6$Alkylene A is, for example, ethylene, propylene, 2,2-dimethylpropylene, tetramethylene or hexamethylene.

If $R_{21}$ and $R_{22}$ together are $C_4$-$C_5$alkylene or -oxaalkylene, this is, for example, tetramethylene, pentamethylene or 3-oxapentamethylene.

The following are examples of polyalkylpiperidine compounds of this class:

70) 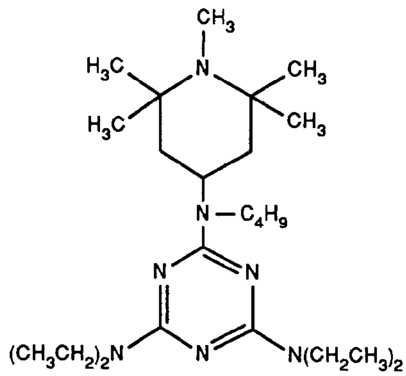

71) 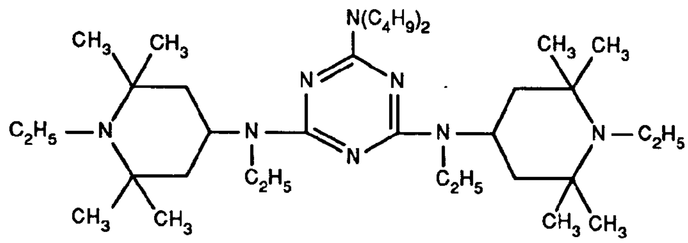

72) 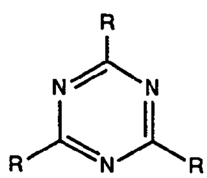 where R = 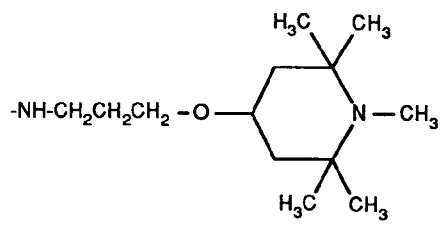

73) 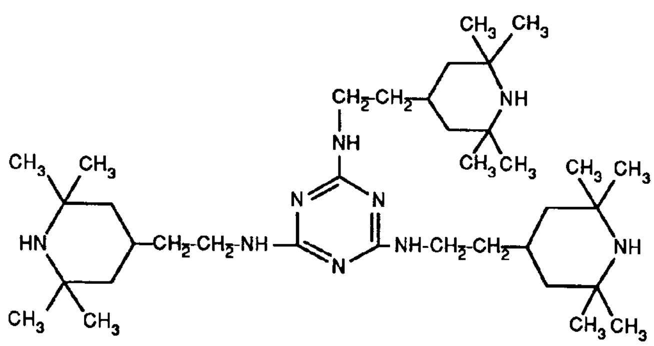

74)

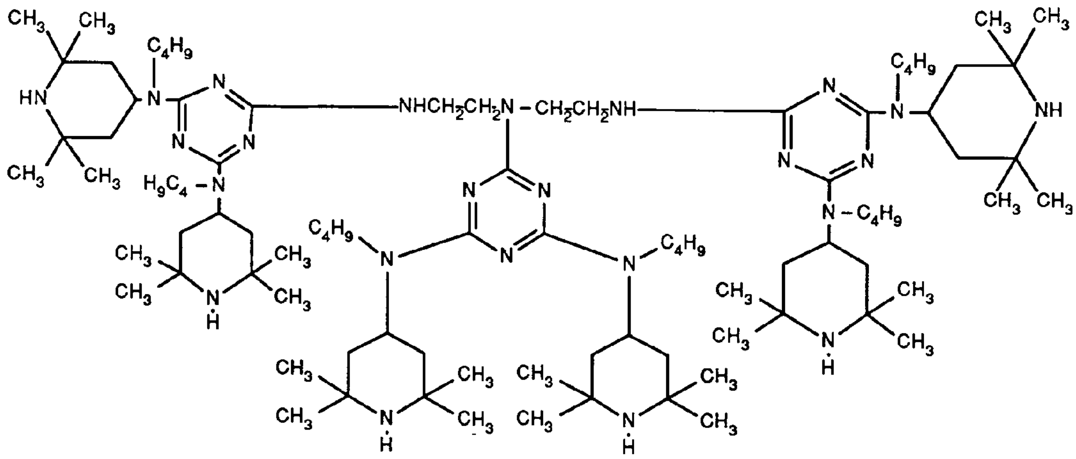

75) 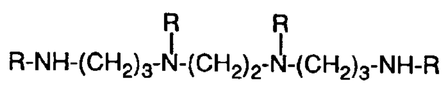

where R = 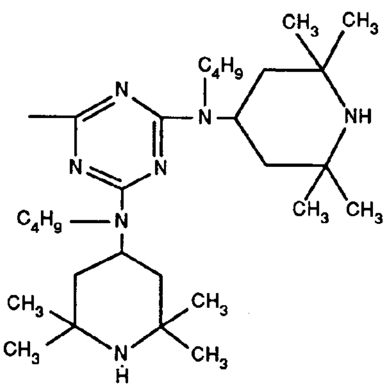

76) 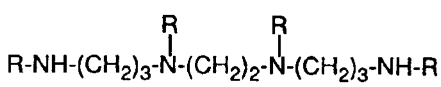

where R = 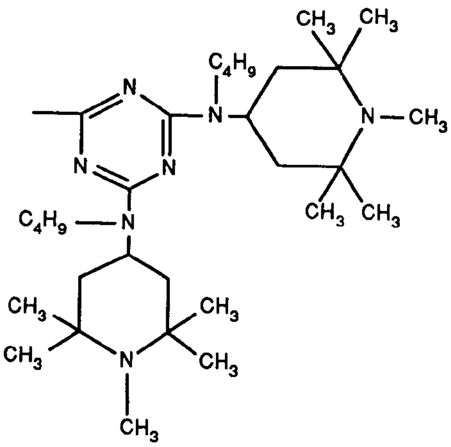

77) 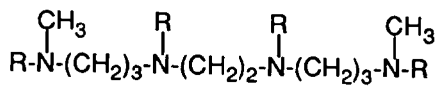

where R = 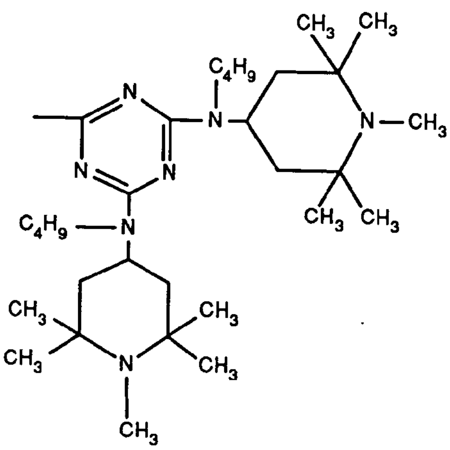

78) 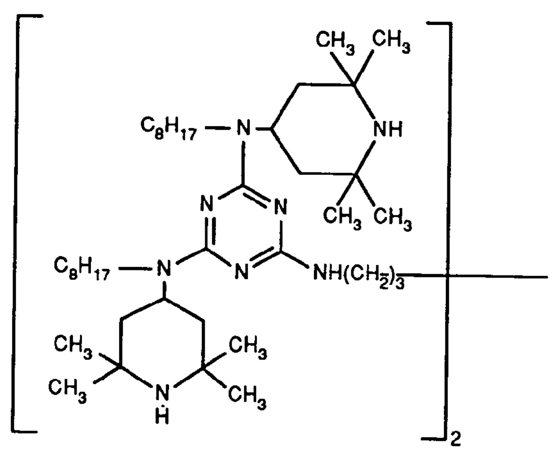

79) 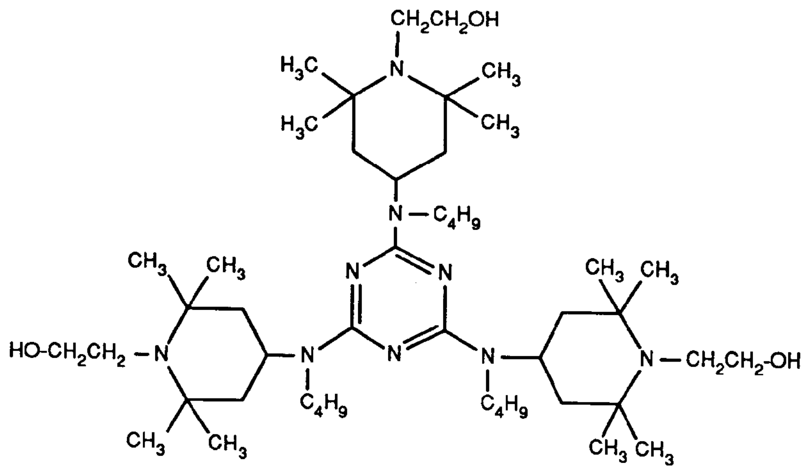

80) 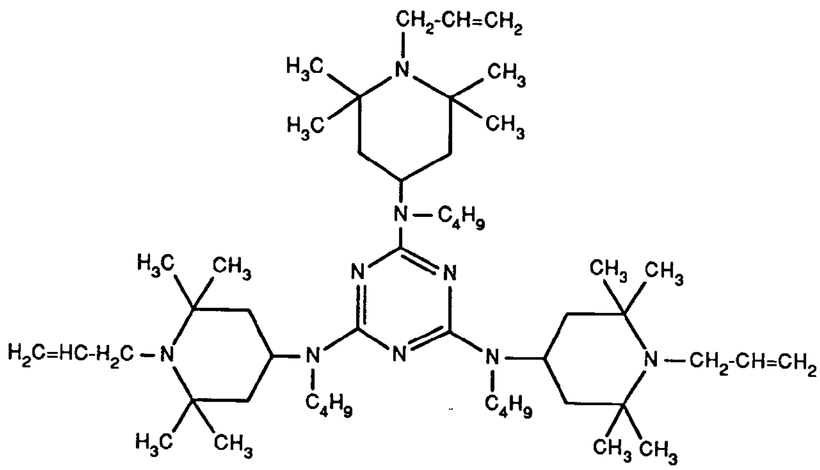

The following are examples of 2,2,6,6-polyalkylpiperidine light screens of this class, where m is a number from 2 to about 200.

81) 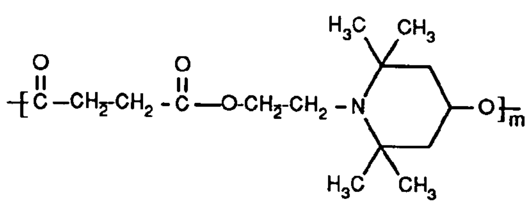

82) 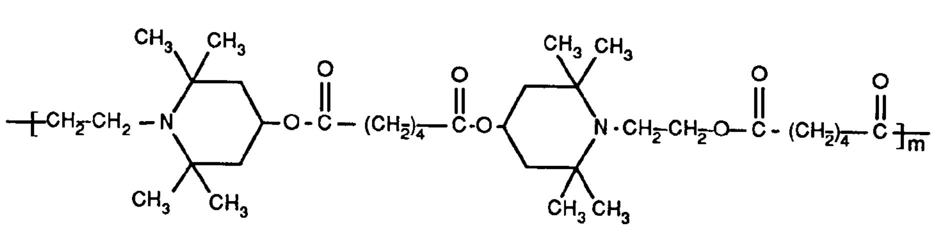

83) 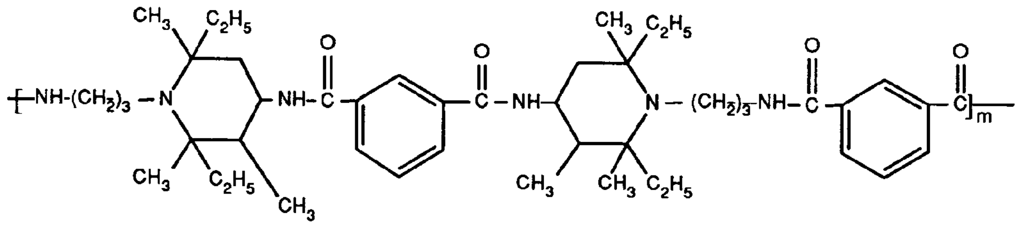

84) 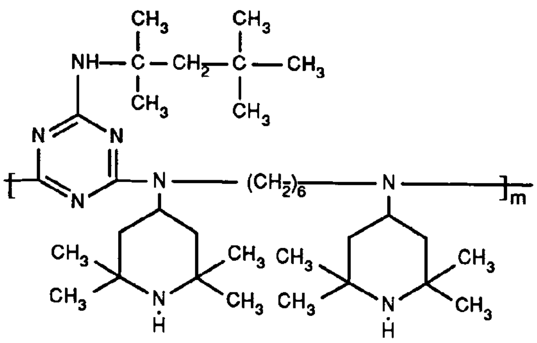

85) 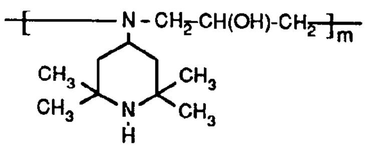

86) 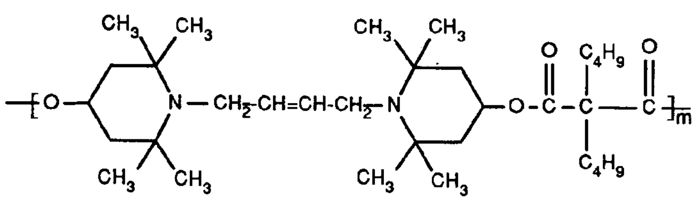

87) 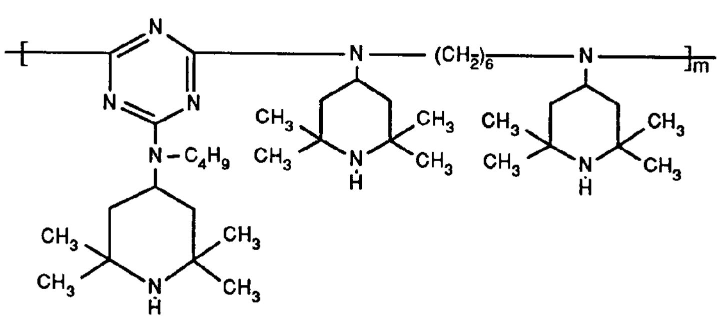

88) 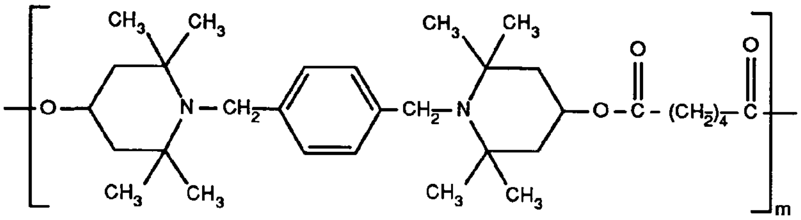

89) 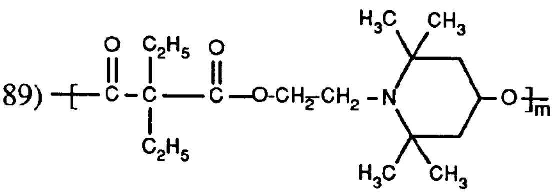

90) 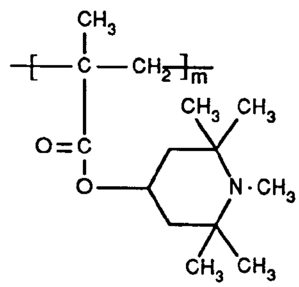

91) 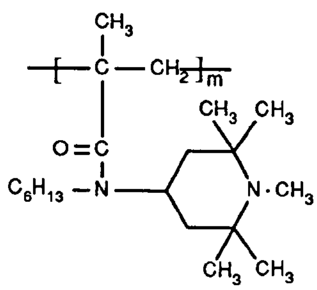

92) 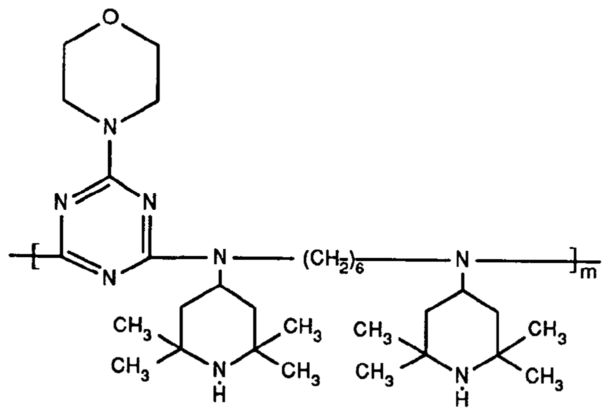

93) 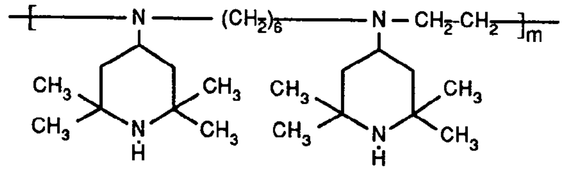

94) 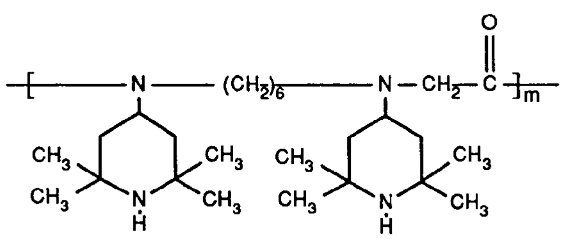

f) Compounds of the formula 6

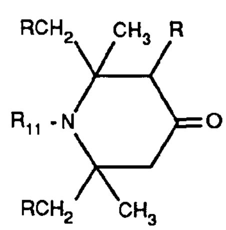

(6)

in which R and $R_{11}$ are as defined under a).

Preferred compounds of the formula 6 are those in which R is hydrogen or methyl, and $R_{11}$ is hydrogen or methyl.

Examples of such compounds are:

95) 2,2,6,6-tetramethyl-4-piperidone (aminotriacetone)

96) 1,2,2,6,6-pentamethyl-4-piperidone

97) 2,2,6,6-tetramethyl-4-piperidone 1-oxide

98) 2,3,6-trimethyl-2,6-diethyl-4-piperidone.

Preferred compounds of formulae I and/or II are

1-tert-butoxy-2,2,6,6-tetramethylpiperidin-4-ol;

bis(1-acetoxy-2,2,6,6-tetramethylpiperidin-4-yl) sebacate;
bis(1-α-methylbenzyloxy-2,2,6,6-tetramethylpiperidin-4-yl) sebacate;
bis(1-octyloxy-2,2,6,6-tetramethylpiperidin-4-yl) sebacate;
bis(2,2,6,6-tetramethylpiperidin-4-yl) sebacate;
1-hydroxy-2,2,6,6-tetramethylpiperidin-4-yl benzoate;
bis(1-oxyl-2,2,6,6-tetramethylpiperidin-4-yl) sebacate;
bis(1,2,2,6,6-pentamethylpiperidin-4-yl) sebacate;
bis(1-hydroxy-2,2,6,6-tetramethylpiperidin-4-yl) sebacate;
bis(1-hydroxy-2,2,6,6-tetramethylpiperidin-4-yl) n-butylmalonate;
bis(1-carbamoyloxy-2,2,6,6-tetramethylpiperidin-4-yl) sebacate;
2,2,6,6-tetramethylpiperidin-4-yl benzoate;
1-oxyl-2,2,6,6-tetramethylpiperidin-4-yl benzoate;
1-formyl-2,2,6,6-tetramethylpiperidin-4-yl benzoate;
1-acetyl-2,2,6,6-tetramethylpiperidin-4-yl benzoate;
bis[1-(2-hydroxyethyl)-2,2,6,6-tetramethylpiperidin-4-yl] sebacate;
1-methyl-2,2,6,6-tetramethylpiperidin-4-yl benzoate;
1-methoxy-2,2,6,6-tetramethylpiperidin-4-yl benzoate;
1-oxyl-2,2,6,6-tetramethylpiperidine;
1-oxyl-2,2,6,6-tetramethylpiperidin-4-ol;
1-hydroxy-2,2,6,6-tetramethylpiperidin-4-ol;
1-α-methylbenzyloxy-2,2,6,6-tetramethylpiperidin-4-yl benzoate;
2,2,6,6-tetramethylpiperidine; or
bis(1-octyloxy-2,2,6,6-tetramethylpiperidin-4-yl) sebacate.

The compounds of formulae I and II are known and can be prepared by methods known per se.

The stabilizers of formulae I and II usually are added in amounts that vary from 0.0001 to 0.1% by weight based on the weight of the organic liquid substrate being stabilized. Preferably the amount of the instant compound is 10 to 500 ppm by weight of the organic liquid being stabilized.

The preferred liquid substrates which are stabilized from oxidation by the instant compounds are light hydrocarbons used in liquid reprographic toners. The use of the instant compounds prevents the formation of odoriferous oxidation products during storage and use under ambient or slightly elevated temperatures.

The prevention of oxidation and of peroxide formation in ethers, such as diethyl ether, and the like is another preferred embodiment of the instant invention.

Preferably the substrate is an organic liquid subject to oxidative or thermal degradation at temperatures below 200°C selected from the group consisting of saturated aliphatic or cycloaliphatic hydrocarbons, oligomeric lower alkylene glycols, aliphatic aldehydes. sulfolane, N-methyl-2-pyrrolidone, lower alkyl nitriles and liquid butadiene rubber.

More preferably the substrate is selected from the group consisting of isooctane, heptane, cyclohexane, tetralin, squalene, polyethylene glycol, polypropylene glycol, polybutylene glycol, sulfolane, N-methyl-2-pyrrolidone, acetonitrile, liquid butadiene rubber and furfural.

Still more preferably, the substrate is tetraethylene glycol, sulfolane, N-methyl-2-pyrrolidone or acetonitrile.

However, the most preferred embodiment of the instant invention is the prevention of oxidation of selective extraction solvents used at temperatures of 100 to 150°C in the refining and separation of petroleum refinery streams. In practice the instant stabilizers can be added to the recycled extraction solvent or to the feed stream at a level of 10 ppb to 500 ppm as a makeup additive to maintain the desired concentration of said stabilizer in the extraction solvent. Typical extraction solvents are the ethylene, propylene or butylene glycol ethers, such as tetraethylene glycol, used in the UDEX process or sulfolane used in a similar process. Most preferably the substrate is the extraction solvent tetraethylene glycol or sulfolane.

The following examples are presented for the purpose of illustration only and are not to be construed to limit the nature or scope of the instant invention in any manner whatsoever.

Example 1: Oxidation of Tetraethylene Glycol

A 1 molar solution of tetraethylene glycol in chlorobenzene is heated at 60°C in the presence of 0.03 mole of 2,2′-azobis(isobutyronitrile), AIBN, free radical initiator, and 0.002 mole of the test stabilizers under an initial 1 atmosphere of oxygen. The rate of oxygen uptake in this AIBN initiated oxidation and final amount of oxygen consumed after 20 hours are measured. A low rate of oxygen uptake and a low total amount of oxygen consumed indicates a more effective antioxidant test stabilizer. The drop in oxygen pressure is a measure of the amount and rate of oxygen uptake.

The results are given on table 1. The results listed are the average of duplicate runs.

Table 1

| Test Stabilizer* | Initial Rate of Oxygen Consumption (mmol/hour) | Total Oxygen Consumption after 20 hours (mmol) |
|---|---|---|
| Blank | 0.09 | 1.47 |
| Compound A | 0.07 | 0.73 |
| Compound B | 0.06 | 0.65 |
| Compound C | 0.04 | 0.53 |
| Compound D | 0.04 | 0.53 |
| Compound E | 0.03 | 0.37 |
| Compound F | 0.01 | 0.37 |
| Compound G | 0.01 | 0.33 |
| Compound H | 0.03 | 0.30 |
| Compound I | 0.01 | 0.30 |
| Compound J | 0.004 | 0.30 |
| Compound K | 0.01 | 0.23 |

*Compound A is 1-tert-butoxy-2,2,6,6-tetramethylpiperidin-4-ol.

Compound B is bis(1-acetoxy-2,2,6,6-tetramethylpiperidin-4-yl) sebacate.

Compound C is bis(1-a-methylbenzyloxy-2,2,6,6-tetramethyl-piperidin-4-yl) sebacate.

Compound D is bis(1-octyloxy-2,2,6,6-tetramethylpiperidin-4-yl) sebacate.

Compound E is bis(2,2,6,6-tetramethylpiperidin-4-yl) sebacate.

Compound F is 1-hydroxy-2,2,6,6-tetramethylpiperidin-4-yl benzoate.

Compound G is bis(1-oxyl-2,2,6,6-tetramethylpiperidin-4-yl) sebacate.

Compound H is bis(1,2,2,6,6-pentamethylpiperidin-4-yl) sebacate.

Compound I is bis(1-hydroxy-2,2,6,6-tetramethylpiperidin-4-yl) sebacate.

Compound J is bis(1-hydroxy-2,2,6,6-tetramethylpiperidin-4-yl) n-butylmalonate.

Compound K is bis(1-carbamoyloxy-2,2,6,6-tetramethylpiperidin-4-yl) sebacate.

When no antioxidant is present, the tetraethylene glycol is rapidly oxidized as seen by the high rate and total amount of oxygen consumed.

While BHT has an intially low rate of oxygen uptake, its antioxidant activity is lost after a few hours and total oxygen consumption is significantly greater than that obtained when an instant compound is used as stabilizer.

Example 2: Oxidation of Sulfolane

Following the same general procedure of Example 1 but replacing tetraethylene glycol with an equivalent amount of sulfolane (tetramethylene sulfone), the antioxidant results obtained are given on table 2 below.

Table 2

| Test Stabilizer* | Initial Rate of Oxygen Consumption (mmol/hour) | Total Oxygen Consumption after 20 hours (mmol) |
|---|---|---|
| Blank | 0.02 | 0.18 |
| Compound F | -0.01 | 0.10 |
| Compound A | 0.01 | 0.10 |
| Compound E | 0.003 | 0.07 |
| Compound H | 0.02 | 0.06 |
| Compound K | 0.008 | 0.06 |
| Compound G | -0.01 | 0.02 |
| Compound I | -0.006 | 0.01 |
| Compound J | -0.001 | 0.01 |

*Compound A is 1-tert-butoxy-2,2,6,6-tetramethylpiperidin-4-ol.

Compound E is bis(2,2,6,6-tetramethylpiperidin-4-yl) sebacate.

Compound F is 1-hydroxy-2,2,6,6-tetramethylpiperidin-4-yl benzoate.

Compound G is bis(1-oxyl-2,2,6,6-tetramethylpiperidin-4-yl) sebacate.

Compound H is bis(1,2,2,6,6-pentamethylpiperidin-4-yl) sebacate.

Compound I is bis(1-hydroxy-2,2,6,6-tetramethylpiperidin-4-yl) sebacate.

Compound J is bis(1-hydroxy-2,2,6,6-tetramethylpiperidin-4-yl) n-butylmalonate.

Compound K is bis(1-carbamoyloxy-2,2,6,6-tetramethylpiperidin-4-yl) sebacate.

Example 3: Oxidation of Saturated Aliphatic Hydrocarbons

Following the general procedure of Example 1, the effectiveness of a number of instant compounds in preventing the oxidation of some selected saturated aliphatic hydrocarbons is measured. The results are given in table 3.

Table 3

| Additive* | Property** | Isooctane | Heptane | Cyclohexane |
|---|---|---|---|---|
| None | OCL-I | 0.5 | 0.5 | 0.8 |
| | OCL-F | 0.5 | 0.5 | 0.8 |
| | Time | 7.7 | 7.6 | 7.3 |
| | Induction | none | none | none |
| | Efficiency | -- | -- | -- |
| Compound L | OCL-I | 0.4 | -- | 0.6 |
| | OCL-F | 0.4 | -- | 0.6 |
| | Time | 13.0 | 9.1 | 14.0 |
| | Induction | none | none | none |
| | Efficiency | -- | -- | -- |
| Compound M | OCL-I | 0.0 | 0.0 | 0.0 |
| | OCL-F | 0.4 | -- | 0.6 |
| | Time | 15.0 | 12.0 | 14.0 |
| | Induction | 3.1 | 3.2 | 4.0 |
| | Efficiency | 2.2 | 2.2 | 2.7 |

*Compound L is 2,2,6,6-tetramethylpiperidin-4-yl benzoate.

Compound M is 1-oxyl-2,2,6,6-tetramethylpiperidin-4-yl benzoate.

**OCL-I is Initial Oxidative Chain Length which is the mols of oxygen consumed per mols of free radical generated.

OCL-F is Final Oxidative Chain Length which is the mols of oxygen consumed after failure per mols of free radical generated.

Time is in hours required to consume 0.1 mmol of oxygen.

Induction is the period in hours till rapid oxygen consumption begins.

Efficiency is the mmol of free radicals trapped per mmol of additive.

The instant compounds are effective antioxidants for retarding the oxidation of isooctane, heptane and cyclohexane.

Example 4: Oxidation of Selected Organic Compounds

Following the general procedure of Example 1, the effectiveness of several instant compounds in protecting tetralin, lauryl aldehyde, polyethylene glycol of molecular weight 600 (PEG), squalene and a liquid butadiene rubber (PBD) from oxidation is measured. The results of these tests are given in table 4.

Table 4

| Additive* | Property** | Tetralin | Lauryl Aldehyde | PBD | PEG | Squalene |
|---|---|---|---|---|---|---|
| None | OCL-I | 10.0 | 105 | 6.8 | 3.0 | 17.0 |
| | OCL-F | 10.0 | 105 | 6.8 | 3.0 | 17.0 |
| | Time | 3.7 | 0.5 | 5.6 | 15.0 | 2.1 |
| | Induction | none | none | none | none | none |
| | Efficiency | -- | -- | -- | -- | -- |
| Compound L | OCL-I | 9.4 | 2.0 | 5.0 | 1.7 | 9.8 |
| | OCL-F | 9.4 | -- | 3.1 | 1.0 | 5.8 |
| | Time | 4.0 | 8.0 | 11.0 | 2.4# | 4.0 |
| | Induction | none | 9.0 | none | none | none |
| | Efficiency | -- | 5.6 | -- | -- | -- |
| Compound M | OCL-I | 7.2 | 2.5 | 3.8 | 0.1 | 6.8 |
| | OCL-F | 7.2 | -- | 3.2 | 0.9 | 6.8 |
| | Time | 5.2 | 8.3 | 13.0 | 6.7# | 35.3 |
| | Induction | none | 9.8 | none | 1.6 | none |
| | Efficiency | -- | 6.3 | -- | 1.1 | -- |

*Compound L is 2,2,6,6-tetramethylpiperidin-4-yl benzoate.

Compound M is 1-oxyl-2,2,6,6-tetramethylpiperidin-4-yl benzoate.

**OCL-I is Initial Oxidative Chain Length which is the mols of oxygen consumed per mols of free radical generated.

OCL-F is Final Oxidative Chain Length which is the mols of oxygen consumed after failure per mols of free radical generated.

Time is in hours required to consume 1.0 mmol of oxygen.

# is time to consume 0.1 mmol of oxygen.

Induction is the period in hours till rapid oxygen consumption begins.

Efficiency is the mmol of free radicals trapped per mmol of additive.

Instant Compound M, the oxyl derivative, is a more effective antioxidant than is the corresponding Compound L.

Example 5: Oxidation of Lauryl Aldehyde

Following the procedure of Example 1, the effectiveness of a number of substituted hindered amine compounds in protecting lauryl aldehyde from premature oxidation at 60°C is measured. The results of these tests are given in table 5.

Table 5

| Additive* | Induction Period in hours | Rate of Oxygen Uptake in (mmole/hour) | Oxidatve Chain Length |
|---|---|---|---|
| None | none | 3.6 | 120 |
| Compound B | none | 2.5 | 93 |
| Compound P | 1.0 | 0.43 | 15 |
| Compound Q | 2.5 | 0.15 | 5.3 |
| Compound R | 9.7 | 0.11 | 3.9 |
| Compound S | 9.8 | 0.08 | 3.4 |
| Compound T | 8.1 | 0.09 | 3.2 |
| Compound L | 9.0 | 0.06 | 2.0 |
| Compound F | 8.3 | 0.05 | 1.9 |
| Compound U | 9.7 | 0.05 | 1.8 |
| Compound E | 15.2 | 0.05 | 1.8 |
| Compound M | 9.8 | 0.07 | 1.5 |

*Compound B is bis(1-acetoxy-2,2,6,6-tetramethylpiperidin-4-yl) sebacate.

Compound E is bis(2,2,6,6-tetramethylpiperidin-4-yl) sebacate.

Compound F is 1-hydroxy-2,2,6,6-tetramethylpiperidin-4-yl benzoate.

Compound L is 2,2,6,6-tetramethylpiperidin-4-yl benzoate.

Compound M is 1-oxyl-2,2,6,6-tetramethylpiperidin-4-yl benzoate.

Compound P is bis[1-(2-hydroxyethyl)-2,2,6,6-tetramethylpiperidin-4-yl] sebacate.

Compound Q is 1-methyl-2,2,6,6-tetramethylpiperidin-4-yl benzoate.

Compound R is 1-methoxy-2,2,6,6-tetramethylpiperidin-4-yl benzoate.

Compound S is 1-oxyl-2,2,6,6-tetramethylpiperidine.

Compound T is 1-a-methylbenzyloxy-2,2,6,6-tetramethylpiperidin-4-yl benzoate.

Compound U is 2,2,6,6-tetramethylpiperidine.

Other compounds useful in this experiment are 1-oxyl-2,2,6,6-tetramethylpiperidin-4-ol and 1-hydroxy-2,2,6,6-tetramethylpiperidin-4-ol.

Example 6

Oxidation of Sulfolane

It is believed that the oxidation of the extraction solvent sulfolane (tetramethylene sulfone) results in the formation of acidic by-products which are probably complex blends of sulfuric, sulfurous and organosulfonic acids. The formation of such acidic products is clearly undesirable as it results in corrosion of processing equipment and the loss of sensitivity of the extraction solvent to selectively extract the petroleum refinery process stream. To measure the effectiveness of various additives on preventing the formation of acidic by-products in the sulfolane process, 50 ml of sulfolane containing 1.5% by weight of water is added to a 250 ml round bottomed flask topped with a condenser, bubbling inlet tube and a stopper. The contents of the flask are heated to 180°C while air is bubbled into the sulfolane at 125 ml/minute. The effluent air carrying any volatile oxidation products is in turn bubbled through 200 ml of water in a trap. After three hours of heating, aliquots from both

the sulfolane and the water trap are titrated to determine acid generated by the oxidation process. The resulting acidity is calculated in micromoles (µm) per gram of sulfolane tested. The results are shown in table 6.

Table 6

| Additive* (ppm) | Total Acidity (µm/g) |
|---|---|
| None | 14.4 |
| Compound V (100) | 8.9 |
| Compound V (50) | 9.2 |
| Compound V (1) | 8.3 |

*Compound V is bis(1-octyloxy-2,2,6,6-tetramethylpiperidin-4-yl) sebacate.

This example demonstrates the reduced formation of acidic products of oxidation as prevented by the presence of low levels of N-hydrocarbyloxy derivatives of hindered amine. The suppression of acidity is even effective at the 1 ppm level. Additionally, the usual formation of brown color and insoluble tar during the oxidation process is also greatly suppressed by the presence of any of the instant hindered amines.

When the corresponding extraction solvent tetraethylene glycol is substituted for the sulfolane, the oxidation of the glycol is also inhibited by the use of low levels of Compound V as evidenced by the reduced formation of tar and acidic products.

## Claims

1. A process for preventing the oxidation of a liquid organic substrate subject to oxidative or thermal degradation at temperatures below 200°C, which process comprises incorporating into said substrate an effective stabilizing amount of a compound from the group of sterically hindered amines, containing at least one moiety of formula I

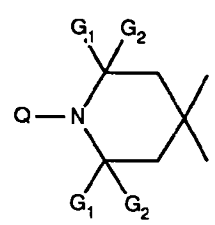

(I)

wherein $G_1$ and $G_2$ are independently alkyl of 1 to 4 carbon atoms, or $G_1$ and $G_2$ together are pentamethylene,
Q is hydrogen, oxyl, hydroxy, alkyl of 1 to 18 carbon atoms, alkenyl of 3 to 18 carbon atoms, alkanoyl of 1 to 18 carbon atoms, alkenoyl of 3 to 18 carbon atoms, phenylalkyl of 7 to 9 carbon atoms, alkanoyloxy of 2 to 18 carbon atoms, alkoxy of 1 to 18 carbon atoms, cycloalkoxy of 5 to 12 carbon atoms, alkenyloxy of 2 to 18 carbon atoms, cycloalkenyloxy of 5 to 12 carbon atoms, phenylalkoxy of 7 to 15 carbon atoms, aryloxy of 6 to 10 carbon atoms or $R_1NHCOO$- where $R_1$ is hydrogen, alkyl of 1 to 12 carbon atoms or phenyl.

2. A process according to claim 1 where in the compound of formula I, $G_1$ and $G_2$ are each methyl.

3. A process according to claim 1 where in the compound of formula I, Q is hydrogen, oxyl, hydroxy, alkyl of 1 to 8 carbon atoms, allyl, alkanoyl of 1 to 8 carbon atoms, alkenoyl of 3 to 4 carbon atoms, benzyl,

alkanoyloxy of 2 to 8 carbon atoms, alkoxy of 1 to 12 carbon atoms, cyclohexyloxy, α-methylbenzyloxy, carbamoyloxy, phenylcarbamoyloxy or alkylcarbamoyloxy of 2 to 9 carbon atoms.

4. A process according to claim 3 wherein Q is hydrogen, oxyl, hydroxy, alkyl of 1 to 4 carbon atoms, allyl, alkanoyl of 2 to 4 carbon atoms, benzyl, acetoxy, alkoxy of 1 to 8 carbon atoms, cyclohexyloxy, α-methylbenzyloxy or carbamoyloxy.

5. A process according to claim 1 wherein the compound, having present the moiety of formula I, is of formula II

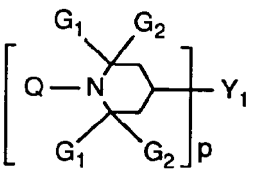

(II)

wherein $G_1$, $G_2$ and Q are defined as described in claim 1,
p is 1 or 2,
when p is 1, $Y_1$ is hydrogen, oxyl, hydroxyl or $-OY_2$ where $Y_2$ is alkanoyl of 2 to 19 carbon atoms, benzoyl or benzoyl substituted by alkyl of 1 to 4 carbon atoms, or
when p is 2, $Y_1$ is $-OCO-Y_3-COO-$ where $Y_3$ is a straight or branched chain alkylene of 2 to 16 carbon atoms.

6. A process according to claim 1 wherein the substrate is an organic liquid subject to oxidative or thermal degradation at temperatures below 200°C selected from the group consisting of saturated aliphatic or cycloaliphatic hydrocarbons, oligomeric lower alkylene glycols, aliphatic aldehydes, sulfolane, N-methyl-2-pyrrolidone, lower alkyl nitriles and liquid butadiene rubber.

7. A process according to claim 6 wherein the substrate is selected from the group consisting of isooctane, heptane, cyclohexane, tetralin, squalene, polyethylene glycol, polypropylene glycol, polybutylene glycol, sulfolane, N-methyl-2-pyrrolidone, acetonitrile, liquid butadiene rubber and furfural.

8. A process according to claim 7 wherein the substrate is tetraethylene glycol, sulfolane, N-methyl-2-pyrrolidone or acetonitrile.

9. A process according to claim 8 wherein the substrate is tetraethylene glycol or sulfolane.

10. A process according to claim 1 wherein the amount of the moiety of formula I is 0.0001 to 0.1% by weight of the organic liquid substrate being stabilized.